# EUROPEAN PATENT APPLICATION

(11) **EP 3 222 230 A1**
(43) Date of publication of application: **27.09.2017**
(21) Application number: 15860544.4
(22) Date of filing: 21.10.2015
(51) Int. Cl.: A61B 17/70, A61F 2/44

(54) **SPACER FOR LAMINOPLASTY**

(30) Priority: 17.11.2014 KR 20140159814
(71) Applicant: CG Bio Co., Ltd., Seongnam-si, Gyeonggi-do 13211 (KR); Jung, Eun Mi, Seoul 03691 (KR)
(72) Inventor: JUNG, Eun-Mi, Seoul 03691 (KR); SEO, Jun-Hyuk, Seongnam-si Gyeonggi-do 13491 (KR); SONG, Dong-Ryul, Yongin-si Gyeonggi-do 16929 (KR); JO, Myoung-Lae, Gwangju-si Gyeonggi-do 12766 (KR); JEONG, Hae-Jun, Seongnam-si Gyeonggi-do 13178 (KR)
(74) Representative: Goddar, Heinz J.
(86) International application number: PCT/KR2015/011129
(87) International publication number: WO 2016/080661

(57) **Abstract**

A spacer for laminoplasty is provided, the spacer including: a first spacer fixed to an upper lamina plate in a state in which an incision surface of the upper laminate plate of a lamina plate of an incised cervical vertebrae is seated on and supported by the first spacer; and a second spacer fixed to a lower lamina plate so that an incision surface of the lower lamina plate (12) of the lamina plate of the incised cervical vertebrae is in contact with and supported by the second spacer, wherein the first spacer and the second spacer are in a coupled state in which they are not separated from each other by means of a fixed bar, while having expandable lengths with respect to each other.

## Description

### TECHNICAL FIELD

The present invention relates to a spacer for laminoplasty, and more particularly, to a spacer for laminoplasty, which includes a first spacer that seats and supports a first incision of a cervical lamina plate and a second spacer that contacts and supports a second incision, the first spacer and the second spacer including a first expansion connection portion and a second expansion connection portion so as to have a mutual ratchet structure and to be coupled to each other in a state in which lengths of the first spacer and the second spacer are expandable with respect to each other, the expansion connection portions providing a function of a bone graft so that a fixation force to the cervical vertebrae can be improved and ease of surgery can be provided.

### BACKGROUND ART

The spine generally includes 24 bones (excluding the sacrovertebral bone), and these bones are connected to one another by joints, and intervertebral discs are located between the joints. These discs absorb shock and provide a significant function of supporting the human position, being a basis of exercise, and protecting the whole internal organs. However, when the spine is injured or twisted due to an external artificial factor, degeneration, or a continuously-maintained abnormal position, which results in compression of nerves that pass through the spinal canal and causes severe pain.

Representative spinal diseases include spinal stenosis, ossification of the posterior longitudinal ligament (OPLL), and the like. Here, the spinal canal is a tubular hollow space in the middle of the spine, and intervertebral foramina occur due to the upper and lower spine, and the middle part of the spinal canal is the space through which nerves (the spinal cord) pass from the brain to limbs. Spinal stenosis is a condition where lumbar pain or several composite nervous symptoms occur due to an abnormal narrowing of the spinal canal in the center of the spine, the nerve root canal or the intervertebral foramina that occur due to any of causes. In this case, a case where the spinal canal of the neck narrows, is called cervical spinal stenosis, and a case where the spinal canal of the waist narrows, is called lumbar spinal stenosis. Also, OPLL is a condition where nerve disorder occurs due to compression of the nerves passing through the spinal canal caused by ossification in which the posterior longitudinal ligament of the spine supported at the rear of the vertebral body and the front of the spinal canal becomes hard like the bone due to several causes.

Generally, laminoplasty procedures concern altering one or more of the bony vertebral structures that surround and define the spinal canal so as to form a wider space in the spinal canal. Laminoplasty is a surgical procedure in which a spacer is placed between anterior and posterior parts of a divided lamina and the separated anterior part and posterior part are stably held and thus the space of the spinal canal is expanded to the maximum.

The above-described spacer for laminoplasty includes a first end fixed to a first incision located at a cervical spinous process, a second end fixed to a second incision located at a cervical transverse process, and a connection portion, which enables a bone graft located between the first end and the second end and fixed to a lower part of the connection portion to be located in an incision space between the first incision and the second incision so that the first end and the second end can be prevented from being moved in a horizontal direction due to an external force.

However, in the conventional spacer for laminoplasty, the first end, the second end, and the connection portion are separately formed in a single plate so that a length of the spacer is not variable and thus is fixed.

Thus, when a situation where an incision distance between the first incision and the second incision is variable, occurs during surgery, if the spacer has a fixed length, as described above, the spacer cannot respond to the incision distance. Thus, there are inconveniences of preparing a spacer having various lengths.

In addition, the spacer has a structure in which a separate bone graft is fixed to the connection portion.

Thus, when the situation where the incision distance between the first incision and the second incision is variable, occurs during surgery, the bone graft having various sizes should be prepared to correspond to the incision distance. Also, there are inconveniences of separately preceding a work for fixing the bone graft.

In addition, because the spacer has a structure in which the first end and the second end are fixed by a screw while being simply in contact with outer surfaces of the first incision and the second incision, when the bone graft has no size corresponding to an incision space, due to separation between an incision surface and the bone graft, when stress occurs in the horizontal direction, a shearing force occurs in the screw of the first end and the second end such that the screw is easily detached from an insertion position when a patient moves.

### DETAILED DESCRIPTION OF THE INVENTION

### TECHNICAL PROBLEM

The present invention provides a spacer for laminoplasty, which includes a first spacer that seats and supports a first incision of a cervical laminoplasty plate and a second spacer that seats and supports a second incision, the first spacer and the second spacer including a first expansion connection portion and a second expansion connection portion so as to have a mutual ratchet structure and to be coupled to each other in a state in which lengths of the first spacer and the second spacer are expandable with respect to each other, the expansion connection portions providing a function of a bone graft so that a fixation force to the cervical vertebrae can be improved and ease of surgery can be provided.

The present invention also provides a spacer for laminoplasty, which includes a first spacer and a second spacer having expandable lengths with respect to each other, being in a coupled state in which they are not separated from each other by means of a fixed bar, wherein the fixed bar is configured in the first spacer and the second spacer to have a more simple structure so that ease of manufacturing can be provided.

Meanwhile, objectives of the present invention are not limited by the above-mentioned objectives, and other unmentioned objectives could be clearly understood by those skilled in the art from the following description.

### TECHNICAL SOLUTION

According to an aspect of the present invention, there is provided a spacer for laminoplasty, the spacer including: a first spacer fixed to an upper lamina plate in a state in which an incision surface of the upper laminate plate of a lamina plate of an incised cervical vertebrae is seated on and supported by the first spacer; and a second spacer fixed to a lower lamina plate so that an incision surface of the lower lamina plate (12) of the lamina plate of the incised cervical vertebrae is in contact with and supported by the second spacer, wherein the first spacer and the second spacer are in a coupled state in which they are not separated from each other by means of a fixed bar, while having expandable lengths with respect to each other.

The first spacer may include: a fixed portion fixed to the upper lamina plate after a screw passes through the fixed portion and then is inserted into the upper laminate plate while being in contact with an upper side of the upper lamina plate; a seating portion configured to be disposed perpendicularly to the fixed portion so that the incision surface of the upper lamina plate is seated on and supported by the first spacer; and an upper expansion coupling portion configured to extend from a connection part of the fixed portion and the seating portion and to be expandably coupled to the second spacer.

The seating portion may include: a seating shelf, which is configured to extend from a fixed plate of the fixed portion to be bent at a predetermined angle and on which the incision surface of the upper laminate plate is seated and supported; and a support flange configured to extend from an end of the seating shelf while having a predetermined angle and to support a part of a lower side of the upper laminate plate.

The upper expansion coupling portion may include: a ratchet piece configured to extend from the fixed plate to be bent at a predetermined angle and having a plurality of ratchets engraved in an upper side of the ratchet piece at predetermined intervals; a guide block formed at a lower side of the ratchet piece; and an expansion hole formed to have an elliptical shape in a longitudinal direction while being perforated in the ratchet piece and the guide block.

The second spacer may include: a fixed portion fixed to the lower lamina plate after a screw passes through the fixed portion and then is inserted into the lower laminate plate while being in contact with an upper side of the lower lamina plate; a lower expansion coupling portion configured to extend from the fixed portion at a predetermined angle and to be expandably coupled to the upper expansion coupling portion of the first spacer; and a contact portion, which is formed in the lower expansion coupling portion and enables the incision surface of the lower laminate plate to be in contact with and supported by the second spacer.

The lower expansion coupling portion may include: a ratchet piece configured to extend from a fixed plate of the fixed portion at a predetermined angle and having a ratchet embossed in a lower side of the ratchet piece; a guide prominent body formed in the lower side of the ratchet piece and providing a guide space in which the upper expansion coupling portion is inserted and guided; and a coupling hole, which is formed through a lower side of the guide prominent body and through which the fixed bar preventing the upper expansion coupling portion inserted into the guide prominent body from being inserted into or detached from the upper expansion coupling portion passes.

The contact portion may include a contact surface, which is formed in the guide prominent body to have an angle perpendicular to the fixed plate and enables the incision surface of the lower lamina plate to be in contact with and supported by the second spacer.

The fixed bar is perforated in the coupling hole from a lower side of the coupling hole of the lower expansion coupling portion so that, in a state in which one end of the fixed bar is located in the guide block of the upper expansion coupling portion inserted into the guide space of the guide prominent body and the expansion hole of the ratchet piece, the other end of the fixed bar is forcibly inserted into or welding-coupled to the coupling hole.

### DESCRIPTION OF THE DRAWINGS

FIG. 1 is a perspective view of a spacer for laminoplasty according to an exemplary embodiment of the present invention;
FIG. 2 is an exploded perspective view of the spacer for laminoplasty of FIG. 1;
FIGS. 3A and 3B are views showing an expanded state of the spacer for laminoplasty of FIG. 1; and
FIG. 4 is a view showing a state in which the spacer for laminoplasty of FIG. 1 is fixed to a cervical vertebrae.

### BEST MODE

Hereinafter, the present invention will be described in detail by explaining an exemplary embodiment of the invention with reference to the attached drawings.

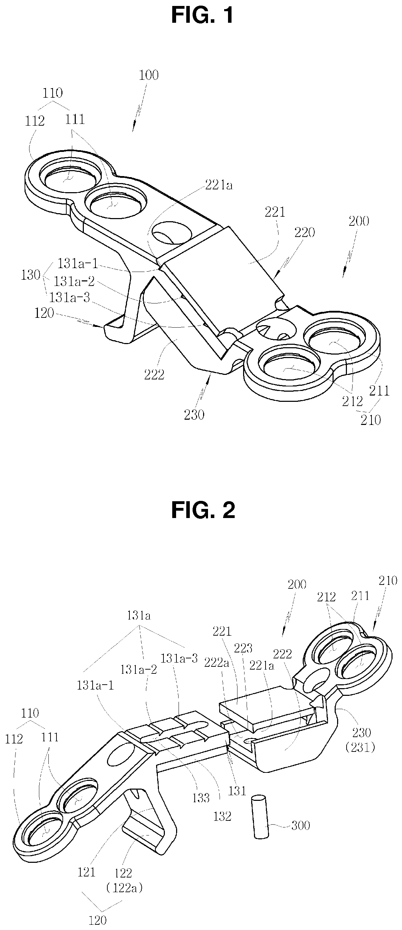

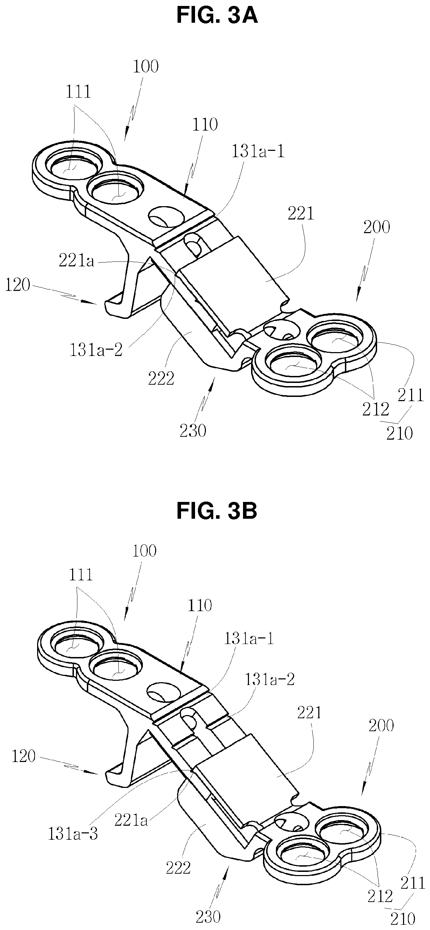

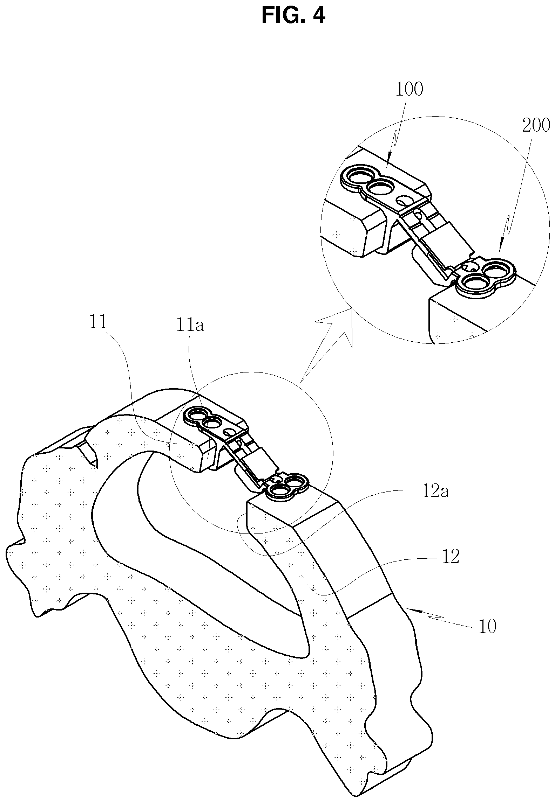

FIG. 1 is a perspective view of a spacer for laminoplasty according to an exemplary embodiment of the present invention, FIG. 2 is an exploded perspective view of the spacer for laminoplasty of FIG. 1, FIGS. 3A and 3B are views showing an expanded state of the spacer for laminoplasty of FIG. 1, and FIG. 4 is a view showing a state in which the spacer for laminoplasty of FIG. 1 is fixed to a cervical vertebrae.

As illustrated in FIGS. 1 through 4, the spacer for laminoplasty according to an exemplary embodiment of the present invention includes a first spacer 100 fixed to an upper lamina plate 11 in a state in which an incision surface 11 a of the upper lamina plate 11 of a lamina plate 10 of an incised cervical vertebrae 1 is seated on the first spacer 100 and supported by the first spacer 100, and a second spacer 200 fixed to a lower lamina plate 12 of the lamina plate 10 of the incised cervical vertebrae 1 so that an incision surface 12a of the lower lamina plate 12 is in contact with the second spacer 200 and supported by the second spacer 200, wherein the first spacer 100 and the second spacer 200 have expandable lengths with respect to each other and are in a coupled state in which they are not separated from each other by means of a fixed bar 300.

As fixed units fixed to an upper lamina plate 11 through a screw inserted into the upper lamina plate 11 in a state in which the incision surface 11 a of the upper lamina plate 11 of the incised cervical vertebrae 1 is seated on the first spacer 100 and supported by the first spacer 100, the first spacer 100 includes a fixed portion 110 fixed to the upper lamina plate 11 after the screw passes through the fixed portion 110 and then is inserted into the upper laminate plate 11 while being in contact with an upper side of the upper lamina plate 11, a seating portion 120, which is disposed perpendicularly to the fixed portion 110 and on which the incision surface 11 a of the upper lamina plate 11 is seated and supported, and an upper expansion coupling portion 130, which extends from a connection part of the fixed portion 110 and the seating portion 120 and is expandably coupled to a lower expansion coupling portion 220 of the second spacer 200 that will be described later.

The fixed portion 110 includes a fixed plate 111 being in contact with the upper side of the upper lamina plate 11, and a fixed hole 112, which is formed in the fixed plate 111 and through which the screw passes. Here, preferably, the fixed plate 111 is used to maximize a contact area by having a width corresponding to a width of an outer circumferential surface of the upper lamina plate 11 and a predetermined length.

The seating portion 120 includes a seating shelf 121, which is disposed perpendicularly to the fixed plate 111 and on which the incision surface 11 a of the upper lamina plate 11 is seated and supported, and a support flange 122, which extends from an end of the seating shelf 121 at a predetermined angle and supports a part of a lower side of the upper lamina plate 11. Here, a support projection 122a protrudes from an end of the support flange 122 by a predetermined height so that the upper side, the incision surface 11 a and the lower side of the upper lamina plate 11 can be more stably fixed and supported by the fixed plate 111, the seating shelf 121, and the support flange 122. Also, the length of the seating shelf 121 may correspond to the thickness of the incision surface 11 a of the upper lamina plate 11.

The upper expansion coupling portion 130 includes a ratchet piece 131, which extends from a connection part of the fixed plate 111 and the seating shelf 121 to be bent at a predetermined angle and has a plurality of ratchets 131 a engraved in an upper side of the ratchet piece 130 at predetermined intervals, a block-shaped guide block 132 formed at a lower side of the ratchet piece 131, and an expansion hole 133, which is formed to have an elliptical shape in a longitudinal direction while the ratchet piece 131 and the guide block 132 pass through the expansion hole 133.

Thus, according to the first spacer 100, when the fixed portion 110 is fixed to the upper lamina plate 11, the incision surface 11 a of the upper lamina plate 11 can be seated and supported by the seating portion 120 so that the fixed portion 110 can be more stably fixed to the upper lamina plate 11.

Here, as the first spacer 100 is formed of a pure titanium material having predetermined elasticity, the length of the seating shelf 121 of the seating portion 120 is larger than the thickness of the incision surface 11 a of the upper lamina plate 11. Thus, even though the upper side of the upper lamina plate 11 has a predetermined distance with respect to the fixed plate 111 of the fixed portion 110 in a state in which the incision surface 11 a and the lower side of the upper lamina plate 11 are supported on the seating shelf 121 and the support flange 122, respectively, the fixed plate 111 of the fixed portion 110 is bendable toward the upper side of the upper lamina plate 11 or is closed so that seating and supporting of the upper lamina plate 11 can be stably performed.

That is, preferably, the first spacer 100 is formed of a pure titanium material having predetermined elasticity. Thus, when a fixing work to the upper lamina plate 11 is performed, a predetermined shape can be changed so that an easier fixing work can be performed.

In addition, unevenness is formed in the fixed plate 111, the seating shelf 121, and the support flange 122 that are in contact with the upper side, the incision surface 11 a and the lower side of the upper lamina plate 11 so that the fixed plate 111, the seating shelf 121, and the support flange 122 can have a more stable seating and contact state.

As fixed units fixed to a lower lamina plate 12 through a screw inserted into the lower lamina plate 12 in a state in which the incision surface 12a of the lower lamina plate 12 of the lamina plate 10 of the incised cervical vertebrae 1 is in contact with the second spacer 200 and supported by the second spacer 200, the second spacer 200 includes a fixed portion 210 fixed to the lower lamina plate 12 after the screw passes through the fixed portion 210 and then is inserted into the lower laminate plate 12 while being in contact with an upper side of the lower lamina plate 12, a lower expansion coupling portion 220, which extends from the fixed portion 210 at a predetermined angle and is expandably coupled to the upper expansion coupling portion 130 of the first spacer 100, and a contact portion 230, which is formed in the lower expansion coupling portion 220 to have an angle perpendicular to the fixed portion 210 and enables the incision surface 12a of the lower lamina plate 12 to be in contact with and supported by the second spacer 200.

The fixed portion 210 includes a fixed plate 211 that is in contact with the upper side of the lower lamina plate 12, and a fixed hole 212, which is formed in the fixed plate 211 and through which the screw passes. Here, preferably, the fixed plate 111 is used to maximize a contact area by having a width corresponding to a width of an outer circumferential surface of the upper lamina plate 11 and a predetermined length.

The lower expansion coupling portion 220 includes a ratchet piece 221, which extends from the fixed plate 211 at a predetermined angle and has a ratchet 221 a corresponding to the ratchet 131 a of the ratchet piece 131 of the upper expansion coupling portion 130 and embossed in a lower side of the ratchet piece 221, a guide prominent body 222, which is formed in the lower side of the ratchet piece 131 and provides a guide space 222a in which the ratchet piece 131 of the upper expansion coupling portion 130 and the guide block 132 are inserted and guided, and a coupling hole 223, which is formed through a lower side of the guide prominent body 222 and to which the fixed bar 300 that maintains a separated state when the guide block 132 of the upper expansion coupling portion 130 is inserted into the guide space 222a of the guide prominent body 222 and the ratchet pieces 131 and 221 are ratchet-coupled to each other.

Here, the fixed bar 300 is perforated in the coupling hole 223 from a lower side of the coupling hole 223 of the lower expansion coupling portion 220 so that, in a state in which one end of the fixed bar 300 is located in the guide block 132 of the upper expansion coupling portion 130 inserted into the guide space 222a of the guide prominent body 222 and the expansion hole 133 of the ratchet piece 131, the other end of the fixed bar 300 is forcibly inserted into or welding-coupled to the coupling hole 223 and thus the embossed ratchet 221 a is in contact with any one of the plurality of engraved ratchets 131 a and mutual ratchet coupling is performed, the state of the embossed ratchet 221 a is not a separated state, i.e., the guide block 132 cannot be inserted into or detached from the guide prominent body 222.

In addition, as the other end of the fixed bar 300 is forcibly inserted or welding-coupled to the coupling hole 223, the one end of the fixed bar 300 does not protrude from the guide block 132 of the upper expansion coupling portion 130 inserted into the guide space 222a of the guide prominent body 222 and an upper side of the expansion hole 133 of the ratchet piece 131 but is located at an inside thereof. Thus, an additional coupling nut does not need to be fastened to the one end of the fixed bar 300 so that the fixed bar has a simply-fixed state.

Meanwhile, in a state in which the fixed bar 300 is formed to have a bolt-shaped screw thread and a bolt-shaped head, the fixed bar 300 may be screw-coupled to the coupling hole 223 having a screw thread in an inner circumferential surface of the fixed bar 300.

Thus, according to the fixed bar 300, the first spacer 100 and the second spacer 200 are coupled to each other while having expandable lengths and then are simply forcibly inserted into, are welding-coupled to or screw-coupled to the coupled space so that a mutually-coupled state thereof cannot be separated and ease of manufacturing can be provided.

The contact portion 230 includes a contact surface 231, which is formed in the guide prominent body 222 of the lower expansion coupling portion 220 to have an angle perpendicular to the fixed plate 211 and enables the incision surface 12a of the lower lamina plate 12a to be in contact with and supported by the second spacer 200.

Thus, according to the second spacer 200, when the fixed portion 210 is fixed to the lower lamina plate 12, the incision surface 12a of the lower lamina plate 12 is seated on and supported by the second spacer 220 by means of the contact portion 230 so that the second spacer 200 can be more stably fixed to the lower lamina plate 12.

Here, as the second spacer 200 is formed of a titanium material having no elasticity, when a fixing work to the lower lamina plate 12 is performed, a fixed position is not changed so that a more stable fixed state can be established.

In addition, unevenness is formed in the fixed plate 211 and the contact surface 231 that are in contact with the upper side of the lower lamina plate 12 and the incision surface 12a so that a more stable seating and contact state can be established.

Hereinafter, an operation and effects of the spacer for laminoplasty having the above-described configuration will be described.

In the spacer for laminoplasty according to the present invention, the first spacer 100 and the second spacer 200 are expandably coupled to each other so that lengths of the first spacer 100 and the second spacer 200 can be changed by means of the upper expansion coupling portion 130 and the lower expansion coupling portion 220. First, the guide block 132 of the upper expansion coupling portion 130 is inserted into an inside of the guide space 222a of the guide prominent body 222 of the lower expansion coupling portion 220 so that the embossed ratchet 221 a formed in the ratchet piece 221 of the lower expansion coupling portion 220 is ratchet-coupled to a first engraved ratchet 131 a-1 of the plurality of engraved ratchets 131 a formed in the ratchet piece 131 of the upper expansion coupling portion 130.

Subsequently, in the above-described state, the fixed bar 300 is perforated in the coupling hole 223 from the lower side of the coupling hole 223 of the lower expansion coupling portion 220 so that one end of the fixed bar 300 is located in the guide block 132 of the upper expansion coupling portion 130 inserted into the guide space 222a of the guide prominent body 222 and the expansion hole 133 of the ratchet piece 131 and then the other end of the fixed bar 300 is forcibly inserted into or welding-coupled to the coupling hole 223 and thus the guide block 132 of the upper expansion coupling portion 130 inserted into the inside of the guide space 222a of the guide prominent body 222 of the lower expansion coupling portion 220 is not inserted in an opposite direction.

Here, when a plurality of engraved ratchets 131 a are formed like a first engraved ratchet 131a-1 to a third engraved ratchet 131 a-3, preferably, ratchet coupling grooves of the engraved ratchet 131 a and the embossed ratchet 221 a are coupled to each other while having an angle at which the engraved ratchet 131 a and the embossed ratchet 221 a are moved only in a direction toward the third engraved ratchet 131 a-3 in a state in which the embossed ratchet 221 a is ratchet-coupled to the first engraved ratchet 131a-1.

Thus, the guide block 132 of the upper expansion coupling portion 130 is inserted into the inside of the guide space 222a of the guide prominent body 222 of the lower expansion coupling portion 220 so that, when the embossed ratchet 221 a formed in the ratchet piece 221 of the lower expansion coupling portion 220 is ratchet-coupled to the first engraved ratchet 131a-1 of the plurality of engraved ratchets 131a formed in the ratchet piece 131 of the upper expansion coupling portion 130, the embossed ratchet 221 a is moved only toward the second engraved ratchet 131 a-2 and the third engraved ratchet 131a-3 so that the lengths of the first spacer 100 and the second spacer 200 can be expanded while having a mutual ratchet structure.

Thus, even when an incision distance between the upper lamina plate 11 and the lower lamina plate 12 is variable during surgery, the upper expansion coupling portion 130 and the lower expansion coupling portion 220 can be expandably (reducibly) coupled to each other while having a ratchet structure so that surgery can be performed simply through expansion or reduction in a site without preparing a spacer having various lengths.

Here, because the upper expansion coupling portion 130 and the lower expansion coupling portion 220 are expandably coupled to each other while having the ratchet structure, as described above, they has a structure in which they are not reduced in a once-expanded state. Thus, even when an external force is applied thereto, a stable expansion state can be maintained. In addition, preferably, a formation distance between the ratchets 131 a and 221 a is 2 mm.

Meanwhile, as described above, the spacer including the first spacer 100 and the second spacer 200 expandably or reducibly coupled to each other while having lengths corresponding to the incision distance between the upper lamina plate 11 and the lower lamina plate 12 through the upper expansion coupling portion 130 and the lower expansion coupling portion 220, is fixed to the upper lamina plate 11 and the lower lamina plate 12.

That is, the fixed plate 111 of the fixed portion 110 of the first spacer 100 and the fixed plate 211 of the fixed portion 210 of the second spacer 200 are fixed to the upper side of the upper lamina plate 11 and the upper side of the lower lamina plate 12, respectively, by inserting the screw. In this case, the incision surface 11 a and the lower side of the upper lamina plate 11 are stably seated on and supported by the first spacer 100 by means of the seating shelf 121 of the seating portion 120 of the first spacer 100 and the support flange 122, and the incision surface 12a of the lower lamina plate 12 is stably in contact with and supported by the contact surface 231 of the contact portion 230 of the second spacer 200.

Thus, the incision surface 11 a of the upper lamina plate 11 and the incision surface 12a of the lower lamina plate 12 can be stably seated on, in contact with, and supported by the first spacer 100 and the second spacer 200, respectively, during surgery without configuring a bone graft filled in the incision distance between the upper lamina plate 11 and the lower lamina plate 12. Thus, a preceding work of separately preparing the bone graft and then coupling the bone graft to the spacer can be omitted, and a state in which a fixing force of the spacer and the lamina plate can be improved.

### INDUSTRIAL APPLICABILITY

Thus, according to the present invention, a first spacer that seats and supports a first incision of a cervical laminoplasty plate and a second spacer that seats and supports a second incision have a mutual ratchet structure and can be coupled to each other by means of a first expansion connection portion and a second expansion connection portion in a state in which lengths of the first spacer and the second spacer are expandable with respect to each other, the expansion connection portions providing a function of a bone graft so that a fixation force to the cervical vertebrae can be improved and ease of surgery can be provided.

In addition, the first spacer and the second spacer having expandable lengths with respect to each other, are in a coupled state in which they are not separated from each other by means of a fixed bar, wherein the fixed bar is configured in the first spacer and the second spacer to have a more simple structure, i.e., in which the first spacer and the second spacer are coupled to each other and then are simply engaged with the coupled space so that ease of manufacturing can be provided.

Meanwhile, the effects of the present invention are not limited by the above-described effects, and other unmentioned effects could be clearly understood by those skilled in the art based on the description of the following claims.

## Claims

1. A spacer for laminoplasty, comprising:
a first spacer (100) fixed to an upper lamina plate (11) in a state in which an incision surface (11 a) of the upper laminate plate (11) of a lamina plate (10) of an incised cervical vertebrae (1) is seated on and supported by the first spacer (100); and
a second spacer (200) fixed to a lower lamina plate (12) so that an incision surface (12a) of the lower lamina plate (12) of the lamina plate (10) of the incised cervical vertebrae (1) is in contact with and supported by the second spacer (200),
wherein the first spacer (100) and the second spacer (200) are in a coupled state in which they are not separated from each other by means of a fixed bar (300), while having expandable lengths with respect to each other.

2. The spacer of claim 1, wherein the first spacer (100) comprises:
a fixed portion (110) fixed to the upper lamina plate (11) after a screw passes through the fixed portion (110) and then is inserted into the upper laminate plate (11) while being in contact with an upper side of the upper lamina plate (11);
a seating portion (120) configured to be disposed perpendicularly to the fixed portion (110) so that the incision surface (11 a) of the upper lamina plate (11) is seated on and supported by the first spacer (100); and
an upper expansion coupling portion (130) configured to extend from a connection part of the fixed portion (110) and the seating portion (120) and to be expandably coupled to the second spacer (200).

3. The spacer of claim 2, wherein the seating portion (120) comprises:
a seating shelf (121), which is configured to extend from a fixed plate (111) of the fixed portion (11) to be bent at a predetermined angle and on which the incision surface (11 a) of the upper laminate plate (11) is seated and supported; and
a support flange (122) configured to extend from an end of the seating shelf (121) while having a predetermined angle and to support a part of a lower side of the upper laminate plate (11).

4. The spacer of claim 2, wherein the upper expansion coupling portion (130) comprises:
a ratchet piece (131) configured to extend from the fixed plate (111) to be bent at a predetermined angle and having a plurality of ratchets (131 a) engraved in an upper side of the ratchet piece (131) at predetermined intervals;
a guide block (132) formed at a lower side of the ratchet piece (131); and
an expansion hole (133) formed to have an elliptical shape in a longitudinal direction while being perforated in the ratchet piece (131) and the guide block (132).

5. The spacer of claim 2, wherein the second spacer (200) comprises:
a fixed portion (210) fixed to the lower lamina plate (12) after a screw passes through the fixed portion (210) and then is inserted into the lower laminate plate (12) while being in contact with an upper side of the lower lamina plate (12);
a lower expansion coupling portion (220) configured to extend from the fixed portion (210) at a predetermined angle and to be expandably coupled to the upper expansion coupling portion (130) of the first spacer (100); and
a contact portion (230), which is formed in the lower expansion coupling portion (220) and enables the incision surface (12a) of the lower laminate plate (12) to be in contact with and supported by the second spacer (200).

6. The spacer of claim 5, wherein the lower expansion coupling portion (220) comprises:
a ratchet piece (221) configured to extend from a fixed plate (211) of the fixed portion (210) at a predetermined angle and having a ratchet (221 a) embossed in a lower side of the ratchet piece (221);
a guide prominent body (222) formed in the lower side of the ratchet piece (221) and providing a guide space (222a) in which the upper expansion coupling portion (130) is inserted and guided; and
a coupling hole (223), which is formed through a lower side of the guide prominent body (222) and through which the fixed bar (300) preventing the upper expansion coupling portion (130) inserted into the guide prominent body (222) from being inserted into or detached from the upper expansion coupling portion (130) passes.

7. The spacer of claim 6, wherein the contact portion (230) comprises a contact surface (231), which is formed in the guide prominent body (222) to have an angle perpendicular to the fixed plate (211) and enables the incision surface (12a) of the lower lamina plate (12) to be in contact with and supported by the second spacer (200).

8. The spacer of claim 6, wherein the fixed bar (300) is perforated in the coupling hole (223) from a lower side of the coupling hole (223) of the lower expansion coupling portion (220) so that, in a state in which one end of the fixed bar (300) is located in the guide block (132) of the upper expansion coupling portion (130) inserted into the guide space (222a) of the guide prominent body (222) and the expansion hole (133) of the ratchet piece (131), the other end of the fixed bar (300) is forcibly inserted into or welding-coupled to the coupling hole (223).
